# EUROPEAN PATENT APPLICATION

(11) **EP 4 850 625 A2**
(43) Date of publication of application: **09.12.2026**
(21) Application number: 26213356.4
(22) Date of filing: 11.10.2023
(51) Int. Cl.: A61B 18/18

(54) **SYSTEMS FOR MICROWAVE ABLATION OF TISSUE**

(30) Priority: 11.10.2022 US 202263414962 P
(62) Document number(s) of the earlier application(s) in accordance with Art. 76 EPC: 23805238.5 / 4 601 573
(71) Applicant: Stryker Corporation, Portage, MI 49002 (US)
(72) Inventor: Peters, Nicolas A., Cork, T12YK68 (IE); Brockman, Christopher S., Kalamazoo, MI, 49008 (US); Andrews, Justin M., Schoolcraft, 49087 (US)
(74) Representative: Schott, Jakob Valentin

(57) **Abstract**

Systems and methods for microwave ablation of tissue. A probe includes a transmission line, and an electrical short provides a choke with a tube. A sheath may be coaxially disposed over the tube and define fluid ports, and a distal infusion path in fluid communication path with a proximal infusion path through an aperture in the tube. The choke may be floating with shorted conductive tubes coaxially disposed opposite a non-conductive tube. The probe may be a double-slot architecture with the choke at a position proximal to the proximal slot. Methods may include providing real-time estimations of tissue condition and/or lesion size. A frequency sweep may be performed from which the relative permittivity of the tissue may be determined. Distances to reflection points of tissue boundaries may be determined in a time domain spectrum, from which a characteristic of the ablation procedure may be provided on a display.

## Description

### PRIORITY CLAIM

This application claims priority to and all the benefits of United States Provisional Patent Application No. 63/414,962, filed on October 11, 2022, the entire contents of which are hereby incorporated by reference.

### BACKGROUND

A microwave ablation procedure uses electromagnetic energy in the microwave frequency regime to heat and destroy tissue. In an oncological setting, the tissue often consists of tumors that generally assume a spheroid shape. The microwave energy is transmitted from a microwave generator to the tissue by a microwave probe, often referred to as an antenna. The energy in the near-field region of the probe interacts strongly with tissue surrounding the probe tip. Owing to the nature of microwave heating, tissue properties influence the performance of the probe. For example, the heating, dehydrating, and charring of the tissue may change its electrical properties, and in particular its relative permittivity at a given frequency of the microwave energy. These changes, if not adequately addressed, may implicate the efficiency and burn pattern of the probe, and may result in uncontrolled and/or non-spherical lesions or ablations. Construction of probes with cooling loops is intricate, thereby adding complexity and cost to manufacturability. Moreover, large amounts of heat are effectively wicked away that could otherwise be utilized to ablate the tissue.

With the ablation occurring inside anatomy, it is difficult to obtain real-time information about the ablation, most notably the size of the lesion. Large changes in the electrical properties of tissue during an ablation may be detrimental to the microwave probe performance and may lead to uncontrolled ablations. Uncertainty about the size of the ablation may result in either insufficient margins of the tumor being destroyed, or collateral damage of healthy tissue adjacent the tumor. Therefore, there is a need in the art for systems and methods to mitigate large changes in the electrical properties of tissue during an ablation and further enable improved real-time estimations of the size of the lesion or other aspects of the ablation procedure.

### SUMMARY

Certain aspects of the present disclosure are directed to mitigating changes in the relative permittivity of the tissue by infusing the tissue with an infusion liquid. The infusion liquid may have a higher relative permittivity to balance the dehydrating of the tissue during the ablation procedure. The infusion liquid is passed through the shaft of the probe prior to infusion in the tissue. Ohmic heating in the shaft may preheat the infusion liquid, which is eventually passed to the tissue. In this way, there is no wasted heat energy in the system. Moreover, the infusion liquid may change state (*i.e.*, from liquid to gas) near the tip of the probe for improved heat transfer. In particular, latent heat energy may be deposited within the tissue when the gas molecules change back to liquid form. It enables faster, larger, and more consistent ablation zones due to active maintenance of the dielectric properties of the tissue without wicking away large amounts of heat energy. Still further, by tuning the flow rate to ensure consistent electrical properties in the tissue immediately adjacent to the tissue, the performance of the probe can be optimized, and therefore an optimal ablation zone achieved. Certain aspects of the present disclosure are also directed to providing improved real-time estimations of tissue condition and of tissue boundaries, and corresponding visualization of the ablation and/or control of the system. Additional advantageous will be readily appreciated from the systems and methods described herein.

The system may include a console, a probe, and optionally a liquid reservoir. The console may include a pump arranged in fluid communication with the liquid reservoir. The console may include a controller in electronic communication with the pump. The probe is configured to be arranged in fluid communication with the liquid reservoir. The probe is further configured to be arranged in operable communication with a microwave generator of the console. The microwave generator is in electronic communication with the controller. The controller is configured to operate the microwave generator to emit the microwave energy at a desired frequency and power.

The probe includes a hub, and a shaft extending distally from the hub to a tip. The shaft may be sufficiently rigid and the tip sufficiently sharp for the probe to pierce and be directed through soft tissue to a target location within the anatomy. The transmission line of the probe may extend from within the hub to within the shaft. The transmission line includes an outer conductor, and an inner conductor coaxially disposed within the outer conductor. An insulative layer is coaxially disposed between the outer conductor and the inner conductor. The transmission line may include a proximal segment, a middle segment, and a distal segment. The inner conductor may extend through the proximal segment and the middle segment, and optionally the distal segment. The distal segment and the middle segment may define a first axial slot, and the middle segment and the proximal segment may define a second axial slot. Such an arrangement may be considered a double-slot architecture. Alternatively, the probe may have a monopolar architecture, or other architectures including single slot, dipole, triaxial, and sleeved, among others.

The probe includes a choke, which may include a tube formed from conductive material and coaxially disposed over a portion of the transmission line. In certain embodiments, the tube may be coaxially spaced apart from the transmission line along its length. An electrical short may provide electrical communication between the transmission line and the tube. The electrical short is formed from conductive material and is in direct contact with each of the outer conductor and the tube. A choke insulative layer is disposed between the tube and the transmission line. A length of the choke defined between the distal end and the electrical short may be one-quarter of the effective wavelength the microwaves at 2.45 GHz in material forming the choke insulative layer, or more generally of the microwave field in the choke insulative layer.

In another embodiment, the choke may not be shorted to the outer conductor of the transmission line. An inner choke layer may be floating, and otherwise spaced apart from of the transmission line. A non-conductive tube is coaxially disposed over a portion of the transmission line. The choke includes the inner choke layer, an outer choke layer, and optionally the electrical short. The inner choke layer includes a first conductive tube coaxially disposed between the non-conductive tube and the outer conductor of the transmission line. The outer choke layer includes a second conductive tube coaxially disposed over the non-conductive tube such that a region of the non-conductive tube forms a choke insulative layer. The region of the non-conductive tube may be thinned to define a recess. The second conductive tube forming the outer choke layer may be disposed within the recess. A length of the region is based on the relative permittivity of the material forming the sheath. The region of the non-conductive tube may have a length approximately equal to one-quarter effective wavelength of a driving frequency in the material of the non-conductive tube.

The electrical short may be a conductive element that is compressed between the outer choke layer and the inner choke layer. The conductive element may be a wire or a ring. The conductive element may be formed from a suitably conductive material such as gold, or alternatively a solder may form the conductive element. The distal end of the inner choke layer terminates at a position proximal to the second axial slot. A distal end of the outer choke layer terminates at a position proximal to the second axial slot.

The probe includes a dielectric housing coupled to the transmission line and defining the tip at a distal end of the probe. The dielectric housing may be bonded to the transmission line, and include an outer diameter at least substantially equal to an outer diameter of the tube. A proximal end of the dielectric housing may abut the distal end of the choke at an interface. The dielectric housing may be coaxially disposed over the proximal segment, and more particularly shaped to contact the outer conductor along its length. The dielectric housing is formed from a material having a relative permittivity and other material properties desirable for percutaneous deployment to within the anatomy.

The probe is configured to deliver the infusion liquid to the tissue surrounding the active tip of the probe, which may be defined at any point along the probe distal to the choke. The probe may include a sheath coaxially disposed over the tube and at least a portion of the dielectric housing. The sheath may be sized to snugly be disposed over the tube and the dielectric housing to define a distal infusion path therebetween. The tube defines a proximal infusion path, and an aperture. The aperture may be a singular hole, a plurality of holes radially arranged about the tube, or the like. The aperture provides fluid communication between the proximal infusion path and the distal infusion path to define the infusion path generally. The aperture may be positioned proximal to the electrical short. An optional barrier may be provided between the sheath and the tube and proximal to the electrical short to avoid backflow of the infusion liquid.

The sheath defines fluid ports in fluid communication with the distal infusion path. The fluid ports may be of any suitable number and arranged in any suitable configuration about the active tip of the shaft as described herein. The fluid ports, or a higher concentration thereof, may be disposed within the active tip at a location to be associated with maximum heating within the ablation zone, also referred to as "hot spots" of the probe. In particular, the location may be proximal to the first axial slot, and/or distal to the second axial slot. Additionally or alternatively, a higher concentration of the fluid ports may be positioned adjacent to the choke than distal to the choke, as the choke may be hot spot. The fluid ports may be of any suitable size or shape.

The controller operates the pump to control characteristics of the delivery of the infusion liquid through the probe and into the tissue. Additionally or alternatively, the controller may operate the pump based on a probe type, and more particularly a tip type corresponding to the characteristics of the fluid ports. Additionally or alternatively, the controller operates the pump based on reflected power as sensed by the microwave generator. The system may also provide for real-time estimations of tissue condition, and real-time estimations of the size of the ablation (or lesion size). The system may also estimate or determine the lesion size in real-time. Such real-time determination of the lesion size may be used to control the flow rate of the infusion liquid, control the delivery of the microwave energy, facilitate proper placement of the probe, and provide data-rich output on the display.

Therefore, a first aspect of the present disclosure is directed to a probe for ablating tissue with microwave energy. The probe includes a transmission line including an outer conductor, an inner conductor coaxially disposed within the outer conductor, an insulative layer coaxially disposed between the outer conductor and the inner conductor. The transmission line defines a first axial slot and a second axial slot located proximal to the first axial slot. A non-conductive tube is coaxially disposed over a portion of the transmission line, and an electrical choke is coupled to the non-conductive tube. The electrical choke includes an inner choke layer, an outer choke layer, and an electrical short. The inner choke layer includes a first conductive tube coaxially disposed between the non-conductive tube and the outer conductor of the transmission line. The inner choke layer has a distal end that terminates at a position proximal to the second axial slot. The outer choke layer includes a second conductive tube coaxially disposed over the non-conductive tube such that a region of the non-conductive tube forms a choke insulative layer. The outer choke layer has a distal end that terminates at a position proximal to the second axial slot. The electrical short provides electrical communication between the inner choke layer and the outer choke layer.

A second aspect of the present disclosure is directed to a probe for ablating tissue with microwave energy. The probe includes a transmission line including an outer conductor, an inner conductor coaxially disposed within the outer conductor, an insulative layer coaxially disposed between the outer conductor and the inner conductor. A non-conductive tube is coaxially disposed over a portion of the transmission line. A region of an outer surface the non-conductive tube defines a recess. An electrical choke is coupled to the non-conductive tube and includes an inner choke layer, an outer choke layer, and an electrical short. The inner choke layer includes a first conductive tube coaxially disposed between the non-conductive tube and the outer conductor of the transmission line. The outer choke layer includes a second conductive tube disposed within the recess such that a portion of the non-conductive tube forms a choke insulative layer. The electrical short provides electrical communication between the inner choke layer and the outer choke layer.

A third aspect of the present disclosure is directed to a probe for ablating tissue with microwave energy. The probe includes a transmission line including an outer conductor, an inner conductor coaxially disposed within the outer conductor, an insulative layer coaxially disposed between the outer conductor and the inner conductor. The transmission line defines a first axial slot and a second axial slot located proximal to the first axial slot. A non-conductive tube is coaxially disposed over a portion of the transmission line. An electrical choke is coupled to the non-conductive tube and includes an inner choke layer, an outer choke layer, and an electrical short. The inner choke layer coaxially disposed between the non-conductive tube and the outer conductor of the transmission line. The outer choke layer is coaxially coupled to an outer surface of the non-conductive tube such that a region of the non-conductive tube forms a choke insulative layer. The outer choke layer has a distal end that terminates at a position proximal to the second axial slot. The electrical short provides electrical communication between the inner choke layer and the outer choke layer. At least one of the inner choke layer, the outer choke layer, and the electrical short are formed through conductive foil and/or electroplating.

A fourth aspect of the present disclosure is directed to a probe for ablating tissue with microwave energy. The probe includes a transmission line including an outer conductor, an inner conductor coaxially disposed within the outer conductor, an insulative layer coaxially disposed between the outer conductor and the inner conductor. A non-conductive tube is coaxially disposed over a portion of the transmission line. An electrical choke is coupled to the non-conductive tube and includes an inner choke layer and an outer choke layer. The inner choke layer including a first conductive tube coaxially disposed between the non-conductive tube and the outer conductor of the transmission line. The inner choke layer is spaced apart from the outer conductor of the transmission line. The outer choke layer includes a second conductive tube such that a region of the non-conductive tube forms a choke insulative layer.

A fifth aspect of the present disclosure is directed to a probe for ablating tissue with microwave energy. The probe includes a transmission line including an outer conductor, an inner conductor coaxially disposed within the outer conductor, and an insulative layer coaxially disposed between the outer conductor and the inner conductor. An electrically conductive tube is coaxially disposed over a portion of the transmission line. An electrical short provides electrical communication between the tube and the outer conductor of the transmission line to define a choke in which a choke insulative layer is disposed between the outer conductor and the tube. The tube defines a proximal infusion path configured to be arranged in fluid communication with a source of infusion liquid, and an aperture. A sheath is coaxially disposed over the tube. The sheath defines a distal infusion path in fluid communication path with the proximal infusion path through the aperture, and fluid ports in fluid communication with the distal infusion path.

A sixth aspect of the present disclosure is directed to a probe for ablating tissue with microwave energy. The probe includes a transmission line including an outer conductor, an inner conductor coaxially disposed within the outer conductor, an insulative layer coaxially disposed between the outer conductor and the inner conductor. The transmission line defines a first axial slot and a second axial slot located proximal to the first axial slot. A tube is coaxially disposed over a portion of the transmission line. An electrical short provides electrical communication between the tube and the outer conductor of the transmission line to define a choke in which a choke insulative layer is disposed between the outer conductor and the tube. A sheath is coaxially disposed over the tube and defines an infusion path configured to be arranged in fluid communication with a source of infusion liquid, and further defines fluid ports positioned between the first axial slot and the second axial slot.

Additional aspects of the present disclosure are directed to implementations of the probe, an instrument console for ablating tissue with microwave energy, and methods of performing an ablation procedure with microwave energy.

### BRIEF DESCRIPTION OF THE DRAWINGS

FIG. 1 is a perspective view of a microwave ablation system including a console, a liquid reservoir, and a probe.
FIG. 2 is an elevation view of the probe.
FIG. 3 is a sectional elevation view of an implementation of the probe of FIG. 2 taken along lines 3-3.
FIG. 4 is a sectional elevation view of another implementation of the probe of FIG. 2 taken along lines 4-4.
FIG. 5A is an enlarged sectional elevation view of the probe in which an infusion flow path includes a proximal flow path and a distal flow path.
FIG. 5B is another enlarged sectional elevation view of the probe in which the infusion flow path further includes a distal fluid port.
FIG. 6 is a schematic representation of electronic aspects of the system in which a switch provides electrical communication between the probe and one of a generator and an analyzer.
FIG. 7 is a plot of S11 versus resonant frequency for the probe at several points during the ablation procedure.
FIG. 8 is a schematic representation of the console being configured to display a real-time estimation of a tissue boundary.
FIG. 9 is an elevation view of another implementation of the probe.
FIG. 10 is a sectional elevation view of a variant of the probe of FIG. 9 taken along lines 10-10.
FIG. 11 is a sectional elevation view of another variant of the probe of FIG. 9 taken along lines 11-11.
FIG. 12A is a sectional elevation view of another implementation of the probe of FIG. 2 taken along lines 12A-12A.
FIG. 12B is a sectional elevation view of another implementation of the probe of FIG. 2 taken along lines 12B-12B.
FIG. 13 is an elevation view of another implementation of the probe in which an inflatable member is coupled to the shaft.
FIG. 14 is a sectional elevation view of a variant of the probe of FIG. 13 taken along lines 14-14.
FIG. 15 is a sectional elevation view of another variant of the probe of FIG. 13 taken along lines 15-15.

### DETAILED DESCRIPTION

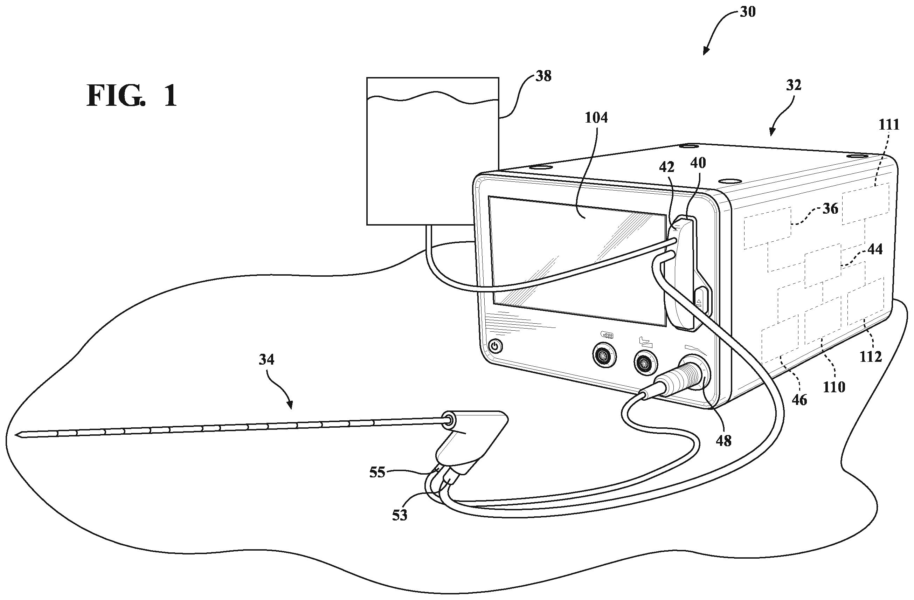

A system 30 for microwave ablation is shown in FIG. 1 in which a console 32 is configured to supply microwave energy to a probe 34. The console 32 may include a pump 36 configured to be arranged in fluid communication with a liquid reservoir 38 to deliver a cooling liquid or an infusion liquid to the probe 34. The console 32 may include a cassette receiver 40 configured to removably receive an irrigation cassette 42 to provide a single-use, sterile barrier between the console 32 and the probe 34. In one example, the irrigation cassette 42 includes tubing, and the pump 36 is a peristaltic pump. The console 32 includes a controller 44 in electronic communication with the pump 36. The controller 44 is configured to operate the pump 36 to control the delivery of the cooling and/or infusion liquid through the probe 34 in manners to be described. Alternatively, the liquid reservoir 38 may be coupled directly to the probe 34 and operable through manual input, or through electromechanical means such as an in-line peristaltic pump coupled with appropriate tubing. In one example, the infusion liquid is liquid saline, but the infusion liquid may be sterile water or another suitable liquid of a known relative permittivity.

The probe 34 is configured to be arranged in fluid communication with the liquid reservoir 38. For example, the probe 34 may include a fluid coupler 53 configured to be coupled to outflow tubing extending from the irrigation cassette 42. The probe 34 is further configured to be arranged in operable communication with a microwave generator 46 of the console 32. The console 32 may include a port 48 for removably receiving an energy coupler 55 associated with a transmission line 50 of the probe 34. The microwave generator 46 is in electronic communication with the controller 44. The controller 44 is configured to operate the microwave generator 46 to emit the microwave energy at a desired frequency and power. In one example, the microwave generator 46 emits the microwave energy at approximately 2.45 Gigahertz (GHz) and at least 150 Watts. The microwave generator 46 may include circuity configured to emit the microwave energy on at least two channels continuously and simultaneously. Other operating parameters of the microwave generator 46 (e.g., frequency, power, channels, etc.) are considered within the scope of the present disclosure.

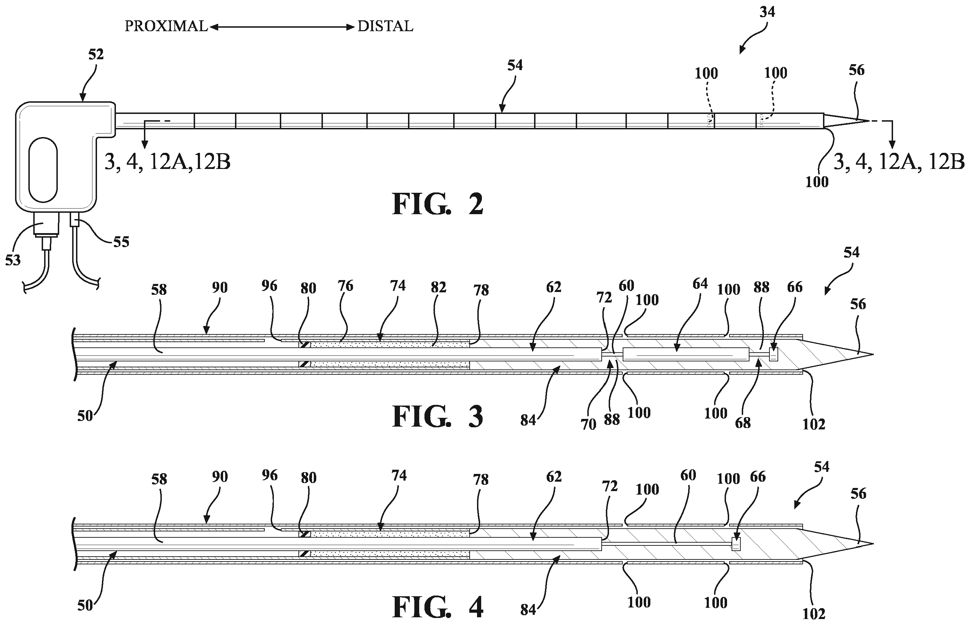

Referring now to FIG. 2, the probe 34 includes a hub 52, and a shaft 54 extending distally from the hub 52 to a tip 56. The hub 52 may include the fluid coupler 53 and the energy coupler 55. The shaft 54 may be sufficiently rigid and the tip 56 sufficiently sharp for the probe 34 to pierce and be directed through soft tissue to a target location within the anatomy. For example, treatment of hepatic lesions may indicate the probe 34 piercing the abdominal musculature to be directed within the liver. Indicia on the shaft 54 may be provided at fixed or varied internals to provide information as to a depth by which the tip 56 has penetrated the tissue.

FIG. 3 shows an implementation of the probe 34 that includes a choked, double-slot architecture. The transmission line 50 of the probe 34 may extend from within the hub 52 to within the shaft 54. The transmission line 50 may be a semi-rigid coaxial cable. The transmission line 50 includes an outer conductor 58, and an inner conductor 60 coaxially disposed within the outer conductor 58. An insulative layer (not identified) is coaxially disposed between the outer conductor 58 and the inner conductor 60. The transmission line 50 may include a proximal segment 62, a middle segment 64, and a distal segment 66. The inner conductor 60 may extend through the proximal segment 62 and the middle segment 64, and optionally the distal segment 66. As such, the inner conductor 60 extends beyond at least a distal end 72 of the proximal segment 62 of the outer conductor 58. The distal segment 66 may be a stub-like portion of the transmission line 50 (i.e., the outer and inner conductors 58, 60 with insulative layer disposed therebetween) with the inner conductor 60 shorted to the outer conductor 58. For example, an exposed distal end of the inner conductor 60 may be deformed and joined to the outer conductor 58 through soldering, swaging, or another suitable joining process. The distal segment 66 of the transmission line 50 may have a length within the range of approximately two to four millimeters, and more particularly approximately three millimeters. Alternatively, the distal segment 66 may be an end cap formed from conductive material and fixedly joined to the inner conductor 60. Another variant is contemplated in which the distal segment 66 may include the stub-like portion of the transmission line 50 to which the end cap is fixedly joined to short the inner conductor 60 to the outer conductor 58.

The distal segment 66 and the middle segment 64 may define a first axial slot 68 characterized by an absence of the outer conductor 58, and the middle segment 64 and the proximal segment 62 may define a second axial slot 70 characterized by an absence of the outer conductor 58. In other words, the segments 62, 64, 66 may be formed by stripping or removing portions of the outer conductor 58 (and optionally the insulative layer) from the inner conductor 60. The size and axial location of the first and second axial slots 68, 70, and consequently the associated sizes and axial locations of the segments 62, 64, 66 are tuned to impart the desired performance characteristics to the probe 34. The size of the first and second axial slots 68, 70 may be the same or different. The first axial slot 68 may have a length within the range of 0.5 to 1.5 millimeters, and more particularly approximately one millimeter. The second axial slot 70 may have a length within the range of 0.5 to 1.5 millimeters, and more particularly approximately one millimeter.

The probe 34 includes a choke 74 to shape near-field properties to facilitate a desired shape of the ablation, preferably a spherical or near-spherical shape. The choke 74 also prevents backward wave propagation along the shaft 54 to limit or prevent undesirable heating of tissue in locations other than the target location. The choke 74 may include a tube 76 formed from conductive material and coaxially disposed over a portion of the transmission line 50. For example, the tube 76 may be a hypodermic tube. With continued reference to FIG. 3, the tube 76 may extend from the hub 52 to a distal end 78 of the choke 74. The tube 76 may be coaxially spaced apart from the transmission line 50 along its length. An electrical short 80 provides electrical communication between the transmission line 50 and the tube 76. More particularly, the electrical short 80 is formed from conductive material and is in direct contact with each of the outer conductor 58 and the tube 76. The electrical short 80 may be ring-shaped and extend annularly between the outer conductor 58 and the tube 76. It is appreciated that the portion of the tube 76 that provides the choke is distal to the electrical short 80, and the tube 76 need not extend rearwardly to the hub 52. Alternatively, the tube 76 may have a length equal to that of the choke 74, and another tube may extend from the hub 52 to provide an infusion path to be described. A choke insulative layer 82 is disposed between the tube 76 and the transmission line 50. The choke insulative layer 82 is formed from non-conductive material and configured to prevent electrical communication between the tube 76 and the outer conductor 58 (other than via the electrical short 80). The non-conductive material has a known relative permittivity. An example of a suitable non-conductive material is polytetrafluoroethylene (PTFE), which has a relative permittivity around 2.2. The choke insulative layer 82 is disposed distal to the electrical short 80, and the choke insulative layer 82 may be coterminous with the tube 76 at the distal end 78 of the choke 74.

The proximal segment 62 may be defined between the distal end 78 of the choke 74 and the second axial slot 70, and the middle segment 64 may be defined between the first and second axial slots 68, 70. As mentioned, the sizes of the segments 62, 64, 66 are tuned to impart the desired performance characteristics to the probe 34. In certain implementations, lengths of each of the proximal segment 62 and the middle segment 64 are one-quarter the effective wavelength of the microwaves at a frequency of 2.45 Gigahertz (GHz) in ablated tissue. Alternatively, the relative lengths of the proximal segment 62 and the middle segment 64 may be different. The resonant length of the middle segment 64 may be within the range of approximately 8 millimeters to 23 millimeters, and more particularly within the range of approximately 8 millimeters to 12 millimeters. A length of the choke 74 defined between the distal end 78 and the electrical short 80 may be one-quarter of the effective wavelength the microwaves at 2.45 GHz in PTFE or other material forming the choke insulative layer 82, or more generally of the microwave field in the choke insulative layer 82.

The probe 34 includes a dielectric housing 84 coupled to the transmission line 50 and defining the tip 56 at a distal end of the probe 34. The dielectric housing 84 may be bonded to the transmission line 50, and include an outer diameter at least substantially equal to an outer diameter of the tube 76. A proximal end of the dielectric housing 84 may abut the distal end 78 of the choke 74 at an interface. The abutting relationship and the complementary outer diameters provide for a smooth transition across the interface. The interface may include a lap joint arrangement in which the dielectric housing 84 defines a recess to receive the PTFE or another adhesive at the interface. An optional seal may be provided at the interface.

The dielectric housing 84 may be coaxially disposed over the proximal segment 62, and more particularly shaped to contact the outer conductor 58 of the first segment 62 along its length. Likewise, the dielectric housing 84 may be coaxially disposed over each of the middle segment 64 and the distal segment 66, and more particularly shaped to contact the outer conductor 58 (or the end cap) of the segment 64, 66 along their lengths. Moreover, inward flanges 88 may surround the inner conductor 60 in each of the first and second axial slots 68, 70. In other words, the dielectric housing 84 may be contoured to a portion of the transmission line 50 that is distal to the choke 74. The dielectric housing 84 may taper to define the tip 56. Certain features of the probe 34 may be disposed within the dielectric housing 84 near the tip 56, for example, sensors for temperature sensing and/or transmitters for intraoperative navigation. In an alternative implementation, the dielectric housing 84 is tubular with a conical tip.

The dielectric housing 84 is formed from a material having a relative permittivity and other material properties desirable for percutaneous deployment to within the anatomy (e.g., stiffness, hardness, ductility, thermal conductivity, electrical conductivity, etc.). An exemplary material is ceramic, and another suitable material is fiberglass. The ceramic may be contoured to the transmission line 50 as described, or the ceramic may be a tube with a thickness sufficient to prevent cracking. The dielectric housing 84 may be formed from a singular material or more than one material. Layers of differing materials may be arranged to result in different relative permittivities at different axial positions along the shaft 54, thereby further shaping near-field properties to facilitate the desired shape of the ablation.

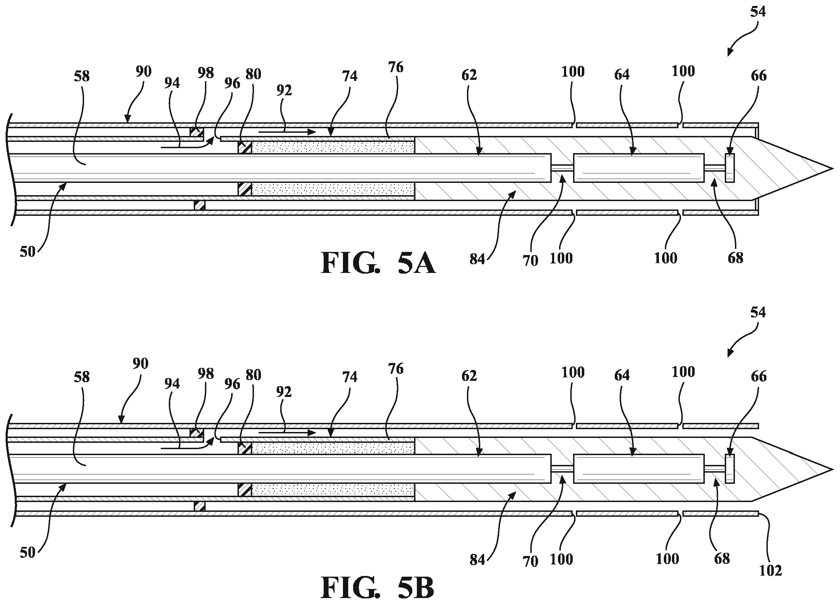

The probe 34 is configured to deliver the infusion liquid to the tissue surrounding the active tip of the probe 34, which may be defined at any point along the probe 34 distal to the choke 74. FIGS. 5A and 5B are enlarged representations of variants of the probe 34 of FIG. 3 to illustrate more clearly the infusion path through the probe 34. The probe 34 includes a sheath 90 coaxially disposed over the tube 76 and at least a portion of the dielectric housing 84. The sheath 90 may be sized to snugly be disposed over the tube 76 and the dielectric housing 84 to define a distal infusion path (arrow 92) therebetween. In one example, the sheath 90 is polymeric, for example, formed from PTFE, wherein the sheath 90 is heat-shrunk onto the tube 76 and the dielectric housing 84. The PTFE is biocompatible, non-stick, and has a melting point above 300 degrees Celsius and therefore well-suited for the ablation procedure. Further, in tubular form, a wall thickness of the PTFE may be selected based on the other parameters of the design of the probe 34 so as to tune the relative permittivity of the probe 34. In another example, the sheath 90 may be a fiberglass tube.

With continued reference to FIGS. 5A and 5B, the tube 76 defines a proximal infusion path (arrow 94), and an aperture 96. The aperture 96 may be a singular hole, a plurality of holes radially arranged about the tube 76, or the like. The aperture 96 provides fluid communication between the proximal infusion path 92 and the distal infusion path 94 to define the infusion path generally. The aperture 96 may be positioned proximal to the electrical short 80. An optional barrier 98 may be provided between the sheath 90 and the tube 76 and proximal to the electrical short 80 to avoid backflow of the infusion liquid. The barrier 98 may be a discrete structure or a location at which the sheath 90 is joined to the tube 76.

The infusion fluid directed to the probe 34 from the liquid reservoir 38 travels along the proximal infusion path 94, through the aperture(s) 96 and along the distal infusion path 92 to be discharged through fluid ports 100 to be described. Advantages of the infusion path of the probe 34 are readily appreciated. First, the proximal infusion path 92 is further defined between the tube 76 and the outer conductor 58 of the transmission line 50. Therefore, as the fluid travels along the proximal infusion path 94, it cools (i.e., wicks away heat from) the transmission line 50. Potential damage to the probe 34 from ohmic heating is minimized. Further, any ohmic heating in the transmission line 50 is used to preheat the infusion liquid, and eventually passed into the tissue. In this way, there is minimal wasted heat energy. Still further, by implementing an open-loop system in which the infusion liquid is directed into the tissue, the effects of latent heat are optionally utilized and the probe 34 may be effectively cooled with a reduced fluid flow (i.e., by utilizing the latent heat of vaporization in the cooling process). Even still further, the infusion preserves the functionality of the choke 74, which may otherwise become compromised if the relative permittivity of the tissue becomes too low. These advantages are realized in a manner that limits the overall size (i.e., outer diameter) of the shaft 54. More particularly, certain structures of the probe 34 (e.g., the choke 74, and the dielectric housing 84) are coaxially arranged about the transmission line 50 that would otherwise interrupt the infusion liquid travelling about the transmission line 50 towards the distal end of the probe 34. By providing the aperture(s) 96 through the tube 76, particularly in combination with the sheath 90 that conforms closely to the outer diameter of the tube 76, cooling of the transmission line 50 is accomplished while still accommodating the other structures of the probe 34 in a space-conscious manner. Stated differently, designing the proximal and distal flow paths 92, 94 with a series of coaxial rigid tubing types is conceivable, but the resulting shaft would be sized too greatly for practical application. In one example, the shaft 54 of the probe 34 may be fourteen gauge or seventeen gauge, but smaller or larger sizes are contemplated.

The sheath 90 defines the fluid ports 100 in fluid communication with the distal infusion path 92. The fluid ports 100 may be of any suitable number and arranged in any suitable configuration about the active tip of the shaft 54. FIG. 2 shows the fluid ports 100 as being equiangularly arranged about the sheath 90 at two axial locations. Additionally or alternatively, the fluid ports 100 may be concentrated on a side of the shaft 54, such as an upper side and/or lower side of the shaft 54 based on, for example, common angles of approach to reach the target anatomy for certain procedures. Additionally or alternatively, there may be one, two (as shown), three, four, five, ten, or twenty or more "rings" of the fluid ports 100 spaced in regular and/or irregular intervals. The fluid ports 100 may be disposed on an entirety of the active tip, or a portion thereof. In one example, the fluid ports 100 may be uniformly distributed radially about the sheath 90, such as along a distalmost two centimeters of the shaft 54. FIG. 5B shows a distal fluid port 102 defined by a distal end of the sheath 90 to discharge the infusion liquid not discharged by the fluid ports 100. FIG. 5A shows a distal barrier 102 configured to prevent discharge of the infusion liquid from near the tip 56.

The fluid ports 100, or a higher concentration thereof, may be disposed within the active tip at a location to be associated with maximum heating within the ablation zone, also referred to as "hot spots" of the probe 34. In particular, the location may be proximal to the first axial slot 68, and/or distal to the second axial slot 70. Additionally or alternatively, the location may be distal to the first axial slot 68, and/or proximal to the second axial slot 70. Additionally or alternatively, the location may be axially aligned with the first and second axial slots 68, 70. Additionally or alternatively, the location may be between the first and second axial slots 68, 70, such as near a center of the middle segment 64 of the transmission line 50. Additionally or alternatively, the location may be along the choke 74. For example, a higher concentration of the fluid ports 100 may be positioned adjacent to the choke 74 than distal to the choke 74, as the choke 74 may be hot spot. The fluid ports 100 may be of any suitable size or shape. In one example, the fluid ports 100 may be relatively larger with a diameter of one millimeter or less. In another example, the fluid ports 100 are formed as perforations in the sheath 90 formed of polymeric material. The fluid ports 100 may be micropores of sufficiently small size such that the shaft 54 may be considered to "weep" the infusion liquid. Lastly, the fluid ports 100 may be the same or different sizes. For example, the fluid ports 100 between the first and second axial slots 68, 70 may be larger than the fluid ports 100 elsewhere.

In another implementation, the fluid ports 100 may be provided at one or more of the locations described herein, and a membrane or filtering layer (not shown) may be disposed over the sheath 90. The filtering layer is configured to disperse the infusion liquid discharged from the fluid ports 100. The filtering layer may provide for a uniform dispersion, or focal dispersion at the hot spots of the probe 34.

Referring now to FIG. 4, another implementation of the probe 34 is shown in which the shaft 54 includes a choked, monopole architecture. With like numerals indicating like components, the inner conductor 60 again extends beyond the distal end 72 of the outer conductor 58. The distal segment 66, such as the end cap, may be fixedly joined to the inner conductor 60. In other words, the transmission line 50 may not include the middle segment 64. A length of the inner conductor 60 between the proximal segment 62 and the distal segment 66 may be one-quarter effective wavelength of ablated tissue. Further, the length of the proximal segment 62 may be one-quarter effective wavelength of ablated tissue. The length of the choke 74 may be one-quarter effective wavelength of the PTFE. The choke 74 and the dielectric housing 84 may otherwise be similar to the implementation previously described.

Likewise, the sheath 90 of the present implementation provides the infusion path for cooling the transmission line 50 and preheating the infusion liquid. The fluid ports 100 may be positioned proximal to the distal segment 66, and/or distal to the proximal segment 62. Additionally or alternatively, the fluid ports 100 may be provided at one or more of the locations previously described herein. As such, it is to be understood that the infusion-related concepts may be extended to antenna types beyond the double-slot and monopole architectures. The present disclosure may be included on other antenna types such as single slot, dipole, triaxial, and sleeved, among others. Moreover, the choke 74 may be an optional feature, wherein the aperture(s) 96 are defined within the tube 76 proximal to the dielectric housing 84 to direct the infusion liquid from the proximal infusion path to the distal infusion path.

The controller 44 operates the pump 36 to control characteristics of the delivery of the infusion liquid through the probe 34 and into the tissue. The characteristics of delivery may be volumetric flow rate, fluid pressure, delivery intervals, among others. The console 32 may include a user interface in communication with the controller 44, such as a display 104 also configured to receive inputs from a user. Therefore, in certain implementations, the user may input a desired flow rate of the infusion liquid, and the controller 44 operates the pump 36 based on the input. For example, the user may input the desired flow rate - in cubic centimeters per minute - and the controller 44 may cause the peristaltic pump to rotate at a speed calibrated to produce the flow rate.

Additionally or alternatively, the controller 44 may operate the pump 36 based on a probe type, and more particularly a tip type corresponding to the characteristics of the fluid ports 100. In other words, the flow rate may be based on the number and/or size of the fluid ports 100 through which the infusion liquid is discharged. For example, the higher number or larger size of the fluid ports 100 may provide for more of the infusion liquid being discharged per unit time. The flow rates for one or more the tip types may be stored as calibration data. The user may input the tip type to the display 104 (or the tip type may be otherwise automatically detected), and the controller 44 operates the pump 36 based on the calibration data. Force feedback on rollers of the peristaltic pump may be sensed, and/or pressure from the pump 36 may be sensed, and the speed with which the pump 36 is operated may be adjusted accordingly in real-time during the ablation procedure. Additionally or alternatively, the controller 44 operates the pump 36 based on reflected power as sensed by the microwave generator 46 in a manner to be described. The responsive control of the pump 36 by the controller 44 may be considered active fluid delivery.

In another implementation, a device (not shown) may be provided for passive fluid delivery. The device may include a mechanism configured to discharge the infusion liquid at a predetermined and constant flow rate. The mechanism may be a spring-loaded plunger, a solenoid valve, or the like. The mechanism may be actuated at or near the commencement of the ablation procedure, after which the device may generally discharge the infusion liquid at the constant flow rate without further user input. The flow of the infusion liquid may be paused or terminated, if desired, for example, by closing the valve or clamping an infusion line. It is also contemplated that the system 30 may provide for the active fluid delivery in combination with the passive fluid delivery. The device may provide the constant flow rate of the infusion liquid at a low level, and the controller 44 may supplement the low level with additional infusion liquid in real-time based on parameters sensed during the ablation procedure or as otherwise indicated by the user.

The advantages of discharging the infusion liquid into the tissue are readily appreciated. First, whereas a closed-loop cooling system can only affect the tissue immediately adjacent the probe through conduction, infusion permits the properties of the tissue to be directly impacted. For example, the effects of dehydration and charring are counteracted, thereby maintaining the relative permittivity of the tissue at the value to which the probe 34 may be optimized. Second, the liquid molecules within the tissue are excitable by the microwave energy from the probe 34. The result may be the infusion liquid adjacent to the probe 34 becoming a functional part of the active tip. Third, the infusion liquid may be heated sufficiently by microwave energy to change state and generate steam. The steam being directed into the tissue provides for additional heat transfer mechanism, which may facilitate achieving the desired ablation zone more quickly and/or a larger ablation zone. Fourth, by routing the irrigation fluid to the ablation site through the ablation probe, the irrigation fluid acts as a coolant to ensure the integrity of the semi-rigid coaxial cable used for high power transfer. This effectively preheats the irrigation fluid prior to deposition in the tissue, as mentioned, and enables a high efficiency system that makes use of lost heating in the coaxial cable.

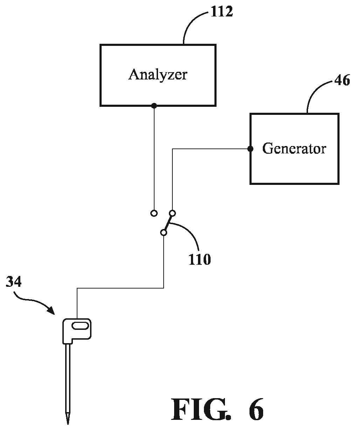

The present disclosure further provides for real-time estimations of tissue condition, and real-time estimations of the size of the ablation (or lesion size). The real-time estimations may be used for controlling the infusion liquid, and/or to present as output to the user on the display 104. For any probe with a fixed resonant length, the resonant frequency is determined by the average relative permittivity of the material with which the near-field interacts. This includes the non-conductive layers of the probe 34, as well at the tissue surrounding the probe 34. While the relative permittivity of the probe materials will stay largely constant throughout the ablation process, the relative permittivity of the tissue may change drastically as the tissue goes from a well-hydrated state at body temperature, to an ablated and dehydrated state. Therefore, the probe 34, if designed to be at resonance for ablated tissue, may be out of resonance for non-ablated or partially ablated tissue. A relationship between the resonant frequency of a simple probe designed to resonate at a quarter wavelength and the average relative permittivity of the surrounding medium may be defined by Equation 1: $f = K \frac{1}{\sqrt{{ε}_{r}}}$ (1) wherein fis resonant frequency, εᵣis the average relative permittivity of the surrounding medium, and Kis a constant equal to c/4Lin which cis the speed of light and Lis the length equal to a quarter wavelength of ablated tissue. Therefore, in a manner to be described, performing a frequency sweep in which an S11 parameter is determined for at least two frequencies, a location of the resonant frequency can be identified, and the average relative permittivity of the tissue may be determined (since the relative permittivity of the probe 34 is fixed). The S11 parameter is based on the reflected power that is reflected back to the microwave generator 46, which may be sensed and quantified. The changes in the average relative permittivity may inform the user of the condition of the tissue, and whether more or less of the infusion liquid should be provided. Moreover, in combination with sensed reflections of the microwaves from tissue interfaces in the time domain spectrum, the lesion size or lesion progress of the ablation procedure may be estimated or determined in real-time. Referring now to FIGS. 1 and 6, the console 32 includes a switch 110, and an analyzer 112. The switch 110 and the analyzer 112 are in electronic communication with the controller 44. The switch 110 may be a high-power, high-isolation switch rated for frequencies up to 20 GHz. The switch 110 is configured to selectively establish electronic communication between the probe 34 and one of the microwave generator 46 and the analyzer 112. More particularly, the switch 110 may be configured to rapidly move the probe 34 between a high-power ablation mode in which the tissue is ablated, and a low-power sensing mode in which the probe 34 functions as a sensor to extract information about tissue condition and lesion size. The analyzer 112 may be a Vector Network Analyzer configured to analyze signals in both the time domain spectrum and the frequency domain spectrum. In one example, the switch may be a quasi-direct current MOSFET, but other switches are contemplated. Alternatively, a filter may be used that includes passive components or distributed filter elements.

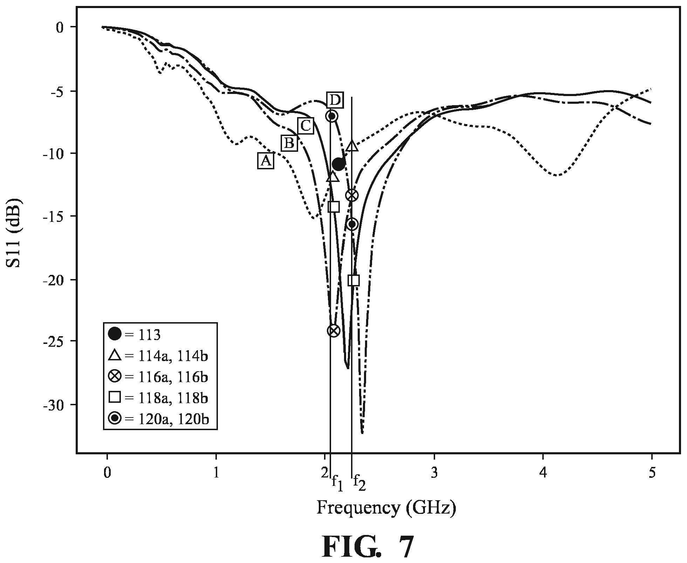

FIG. 7 shows traces of the S11 parameter as a function of frequency for an implementation of the probe 34 at several points during the ablation procedure (i.e., each trace characterizes a different point in the procedure). In particular, Trace A may represent a state or condition at the start of the ablation procedure in which the resonant frequency is less than 2 GHz. As the microwave energy is delivered to the tissue with the probe 34, the tissue is ablated and loses water content, leading to a drop in the average relative permittivity (εᵣ). Because the resonant frequency is proportional to the inverse square root of the average relative permittivity (see Equation 1), the resonant frequency correspondingly shifts from lower to higher frequencies, as reflected in Traces B, C, and D. Since it is preferable for the microwave energy to be radiated into the tissue as opposed to being reflected back to the microwave generator 46, it is desirable for the resonant frequency to match the driving frequency for the majority of the ablation time (e.g., Trace D at approximately 2.3 GHz).

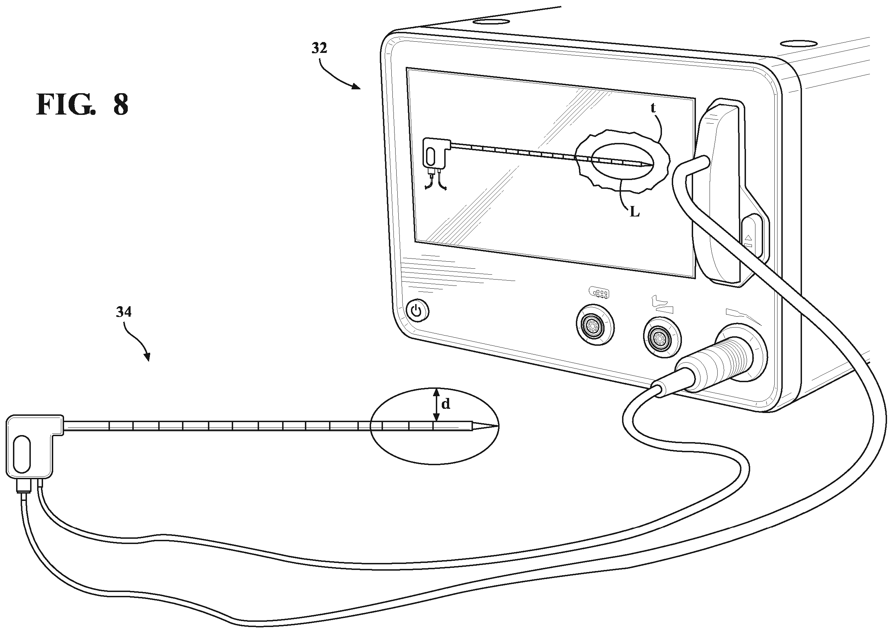

A method of performing the procedure includes delivering the microwave energy from the microwave generator 46 to the probe 34 to ablate the tissue. Memory 111 in electronic communication with the controller 44 stores calibration data including a plurality of "power curves," for example, the traces of FIG. 8. Each of the power curves may include data indicative of the resonant frequency associated with each of different known values of the average relative permittivity for the probe 34 as designed. The average relative permittivity may be a weighted average relative permittivity since tissue near the probe 34 has a greater impact on the average relative permittivity than tissue farther away from the probe 34. The switch 110 may be in a first position in which electronic communication is established between the microwave generator 46 and the probe 34. A measured S11 parameter 113 may be determined based on reflected power sensed by the microwave generator 46 at the effective or driving frequency. The controller 44 may actuate the switch 110 to a second position in which electronic communication is established between the probe 34 and the analyzer 112. In doing so, the probe 34 may be moved from the high-power ablation mode at which the microwave energy is delivered to the probe 34 at a first wattage, to the low-power sensing mode at which the frequency sweep is performed at a second wattage that is less than the first wattage. The frequency sweep is performed by either the microwave generator 46 within its operable range, or by the analyzer 112 in which a sensed S11 parameter is determined for at least two frequencies within a frequency range. By actuating the switch 110 to the analyzer 112, the frequency range may be outside an operable range of the microwave generator 46, thereby providing robust datapoints for analysis. FIG. 8 identifies two examples of frequencies (f₁, f₂) of the frequency range.

At least two datapoints of the sensed S11 parameter are determined with each of these two datapoints associated with a respective one of the two frequencies. The two datapoints of the sensed S11 parameter are evaluated in view of the calibration data for the probe 34. From the two datapoints of the sensed S11 parameter, one of the power curves may be selected as indicative of a state of the ablation procedure. In other words, the two datapoints may uniquely correspond to one of the power curves. For example, at the start of the ablation procedure, the two datapoints 114a, 114b of the sensed S11 parameter may be determined, and the controller 44 determines that Trace A characterizes the weighted average relative permittivity of the medium (i.e., the probe 34 within the tissue) at or around that moment in time based on the performance of the probe 34 within the medium. Only one datapoint may be insufficient to select the appropriate power curve, as more than one of the power curves may be associated with certain datapoints.

The method may include determining a slope of the datapoints. The slope may be either positive or negative. Given the nature of the power curves, the slope may be indicative of whether the resonant frequency for the corresponding power curve (also referred to herein as probe resonant frequency) is below, at or above the driving frequency. This determination may provide a characteristic of the ablation procedure based on the measured S11 parameter 113 and the frequency sweep. More particularly, the determined characteristic may inform the system 30 as to whether the tissue condition is progressing as desired, or perhaps the tissue has begun dehydrating, desiccating, or charring such that the controller 44 should commence or increase the flow rate of the infusion liquid (and/or adjust the delivery of the microwave energy). Further, the extent by which the frequency of the measured S11 parameter 113 is below, at, or above the resonant frequency may be indicative of a magnitude by which the corrective or corresponding actions should be taken.

By way of examples and with continued reference to FIG. 8, the two datapoints 114a, 114b for Trace A include a positive slope. Owing to the general shape of the power curves, since the determined slope is positive, it can be assumed that the probe resonant frequency is less than the driving frequency, and consequently the weighted average relative permittivity is too high for the probe 34 to be operated optimally for the driving frequency. Stated simply, the determined characteristic may be that the tissue is in a non-ablated state. As a result, the controller 44 may operate the pump 36 to reduce the flow rate of the infusion liquid provided to the tissue (and/or adjust the delivery of the microwave energy). Further, the magnitude of difference between the measured S11 parameter 113 and the current resonance frequency may be determined, and the controller 44 may operate the pump 36 based on the determined magnitude. In one example, the controller 44 may not initiate or increase the flow rate of the infusion liquid until the probe resonant frequency matches the driving frequency. In another example, the controller 44 may initiate, decrease, or increase the flow rate of the infusion liquid by an extent proportional to the difference between the probe resonant frequency and the driving frequency such that overly dehydrated tissue more quickly receives the infusion liquid. The tissue condition may be displayed on the display 104, either quantitatively or qualitatively. For example, the quantitative output may include displaying the relative permittivity of the tissue, and the qualitative output may include displaying color coding or other indicia indicative of the tissue condition.

Depending on the selected power curve, control algorithms may forego initiating or increasing the flow rate of the infusion liquid if the determined slope of the datapoints is positive. One instance may be situations in which the probe resonant frequency is less than the driving frequency of the probe 34. In such an example, the weighted average relative permittivity of the selected power curve may be too high (relative to the weighted average relative permittivity for which the probe 34 is designed). Stated differently, the probe 34 is optimized for the tissue to be in an ablated condition with some tissue dehydration. In the illustrated example of FIG. 7, Trace A may provide such a scenario. The resonant frequency for Trace A is less than the driving frequency, and therefore the S11 parameter at the driving frequency is undesirably high such that too much of the microwave energy is being reflected to the microwave generator 46. Therefore, if the selected power curve is Trace A, the controller 44 may reduce pump 36 operation if the two datapoints 114a, 114b associated with Trace A have a positive slope in order to drive the power curves towards Traces B, C, and/or D.

The determined slope of the two datapoints of the sensed S11 parameter may be negative. Trace D from FIG. 7 includes two datapoints 120a, 120b at the two frequencies of the frequency sweep. Based on the negative slope, the controller 44 may operate the pump 36 to increase the flow rate of the infusion liquid provided to the tissue (and/or adjust the delivery of the microwave energy). With further delivery of the microwave energy and with more of the infusion liquid being infused, dehydration of the tissue may be reversed such that the weighted average relative permittivity of the medium is increased to correspond to the weighted average relative permittivity for which the probe 34 is designed. Eventually, the probe resonant frequency will be near or equal to the driving frequency for the microwave generator 46, which corresponds to the least reflected power and optimal operation of the probe 34. If the probe resonant frequency is equal to the driving frequency for the microwave generator 46, the controller 44 may operate the pump 36 to maintain the flow rate of the infusion liquid (or increase or decrease if otherwise necessary).

In addition to the tissue condition, it may be desirable to estimate or determine the lesion size in real-time. Such real-time determination of the lesion size may be used to control the flow rate of the infusion liquid, control the delivery of the microwave energy, facilitate proper placement of the probe 34, and provide data-rich output on the display 104, among other advantages. As mentioned, the analyzer 112 may be configured to analyze signals in both the time domain spectrum and the frequency domain spectrum. The method may include transforming the frequency sweep from the frequency domain spectrum to the time domain spectrum. The time domain spectrum may be considered to show the time-variant response to a Gaussian-like electric pulse, whereas the frequency domain spectrum splits the waveform into individual frequency components. The step of transforming the frequency sweep from the frequency domain spectrum to the time domain spectrum may include applying Fourier transforms.

Interfaces between tissues of differing electrical properties reflect the microwave energy in the time domain spectrum. Exemplary interfaces include lesion boundary, tumor boundary, and boundaries of liver, veins, arteries, and other anatomical structures. Measuring the reflections in the time domain spectrum may be used to determine the lesion size, and therefore the progress of the ablation procedure. The relationship between the group velocity of electromagnetic radiation in a media and the relative permittivity of the media is shown in Equation 2: ${c}_{eff} = \frac{c}{\sqrt{{ε}_{r}}}$ (2) wherein c is the velocity of light, εᵣis the average relative permittivity of the surrounding medium, and *c_{eff}* is effective speed of the microwaves in the tissue. As the weighted average relative permittivity of the medium drops, the effective speed of the microwaves increases. An increase in the effective speed of the microwaves reduces the time by which the microwaves are emitted by the probe 34, reflected by any given interface in the tissue, and returned to the probe 34. As the interface forms or shifts between ablated and non-ablated tissue (*e.g.*, coagulated and non-coagulated tissue), the change in the reflection time can be sensed and determined.

Exemplary methods include the steps of determining the weighted average relative permittivity based on the resonant frequency of the medium, for example, the manner previously described. The speed of the microwaves may then be determined based on the weighted average relative permittivity and sensed times by which the microwaves are reflected in the tissue. As mentioned, changes in the weighted average relative permittivity, as determined by the frequency sweep, affects the speed of the microwaves through the medium. A change in the speed of the microwaves may then be determined from the frequency domain data. Based on the speed of the microwaves and the resonant frequency, reflection points from the interfaces may be determined or "visualized." In other words, distances to the interfaces from the probe 34 can be determined. The distances may be compared to previous determined distances to determine a presence or absence of one or more of the reflection points. The presence of a new reflection point, for example, may be indicative of a new interface forming or shifting at the lesion boundary. It is also appreciated that the signals in the frequency and time domains spectrums, in combination, can also be used to determine the average relative permittivity along the path of the microwave energy, which is a measure of tissue ablation.

Based on the determined distances, the size of the lesion may be approximated. Based on the size of the lesion with other parameters of the ablation procedure, the controller 44 may react in a corresponding manner. For example, the delivery of the microwave energy may be terminated if it is determined the probe 34 is at or has passed through the tumor boundary and into adjacent, healthy tissue. Additionally or alternatively, the controller 44 may operate the pump 36 to increase or decrease the flow rate of the infusion liquid.

From the aformentioned methods, data-rich output may be provided on the display 104. For example, an indication may be displayed to alert the user that the probe 34 has passed through the interface between the tumor and the adjacent tissue, either to facilitate placement of the probe 34 or warn of improper placement. Additionally or alternatively, graphics may be displayed on the display 104 in which a representation of an estimated lesion size is provided. The representation may be two-dimensional, three-dimensional, or an animation, and further visual representations may include lines or shapes to virtually map the placement of the probe 34 and the corresponding ablation zone. FIG. 8 shows one such example in which the representation of the estimation lesion size (L) is overlaid on preoperative imaging, e.g., a computed tomography (CT) scan in which the tumor (t) and other tissue boundaries may be identified. As mentioned, a distance (d) to the interface(s) from the probe 34 can be determined based on the reflection points. Further, the CT scan may be segmented to extract features, and the controller 44 may correlate the reflection signals with the extracted features from the CT scan. Based on the correlation and the real-time estimation of the tissue condition and/or the lesion size, the controller 44 may adjust operation of the system 30 accordingly (e.g., increase or decrease the infusion liquid and/or the microwave energy). Further, the CT scan may be analyzed digitally, from which the initial parameters of the ablation procedure may be automatically determined. Based on the initial parameters and the real-time estimations, the controller 44 may adjust operation of the system 30 accordingly. In other words, the controller 44 may reconcile, intraoperatively, current parameters with the initial parameters if the ablation procedure is deviating too greatly or progressing differently than as planned.

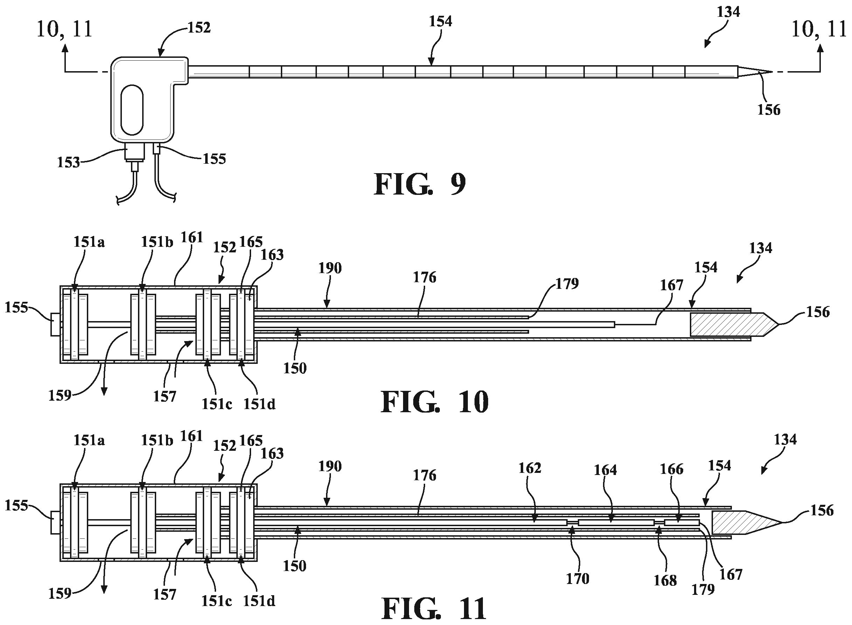

The implementations of the probe 34 discussed thus far discharge the infusion liquid into the tissue. Certain implementations of the probe may be, additionally or alternatively, configured to circulate the liquid, wherein such liquid may be considered cooling liquid. Referring now to FIGS. 9-11, implementations of a probe 134 are shown in which like numerals, plus one hundred, indicate like components. The probe 134 includes the hub 152, and the shaft 154 extending distally from the hub 52 to the tip 156. The tip 156 may be a short, discrete structure coupled to only the sheath 190, as shown, or may be formed by a dielectric housing disposed about the transmission line 150 similar to implementations previously described. The hub 152 may include the fluid coupler 153 and the energy coupler 155. FIGS. 10 and 11 show schematic representations of the hub 152 in which the fluid coupler 153 includes a fluid inlet 157 and a fluid outlet 159, and the energy coupler 155 is a fitting.

The hub 152 may include an outer housing 161, and fluid barriers 151 disposed within the outer housing 161. The fluid barriers 151 are axially spaced apart from one another and coupled to certain subcomponents of the probe 134 as to be described to define an inflow path and an outflow path that is fluidly separate from the inflow path. Each of the fluid barriers 151 may include at least one sealing member 163 fixed to an inner surface of the outer housing 161, and a seal 165 such as an O-ring. More particularly, a first of the fluid barriers 151a may be positioned proximal to the fluid outlet 159 and configured to prevent egress of fluid through the fitting 155. A second one of the fluid barriers 151b may be positioned between the fluid outlet 159 and the fluid inlet 157. The second fluid barrier 151b defines a bore through which the transmission line 150 extends for the transmission line 150 to be in electrical communication with the fitting 155. A distal surface of the second fluid barrier 151b may be coupled to the tube 176 coaxially disposed about the transmission line 150 and extending into the shaft 154. The tube 176 may be formed from polyamide or other suitable material. The second fluid barrier 151b may fluidly separate the inflow path and the outflow path. A third of the fluid barriers 151c may be positioned distal to the fluid inlet 157. The third fluid barrier 151c may define one or more bores through which the transmission line 150 extends, and through which the tube 176 extends. A distal surface of the third fluid barrier 151c may be coupled to the sheath 190 coaxially disposed about the transmission line 150 and the tube 176. The sheath 190 may be PTFE, as previously described, or fiberglass tubing. A fourth of the fluid barriers 151d may be positioned distal to the fluid inlet 157 and configured to prevent egress of fluid through the outer housing 161 adjacent to the shaft 154.

FIG. 10 shows the antenna type as a monopole architecture. The tube 176 extends from the hub 152 to a distal end 179 of the tube 176. The distal end 179 of the tube 176 may be axially positioned proximal to a distal end 167 of the transmission line 150. Alternatively, the distal end 179 may be coterminous with or axially positioned distal to the distal end 167 of the transmission line 150. The cooling liquid is directed through the fluid inlet 157 and into an annular space defined between the tube 176 and the sheath 190. The cooling liquid may consume the space proximal to the tip 156 and about the transmission line 150. Since the inflow path is spaced apart from the transmission line 150 until closer to its distal end 167, maximum cooling effect is provided to the portion of the transmission line 150 ablating the tissue. The tissue itself is also cooled, providing some degree of control over the electrical properties of the tissue. The cooling liquid is directed through the outflow path further defined between the tube 176 and the transmission line 150, and then through the fluid outlet 159 of the hub 152.

FIG. 11 shows the antenna type as the double-slot architecture. The distal end 179 of the tube 176 is axially positioned coterminous with the distal end 167 of the transmission line 150. Alternatively, the distal end 179 may be axially positioned proximal or distal to the distal end 167 of the transmission line 150, and more particularly proximal to the first axial slot 168, the second axial slot 170, or any position therebetween. The first axial slot 168 and the second axial slot 170 are defined between the proximal, middle, and distal segments 162, 164, 166, as previously described. The cooling liquid is directed through the fluid inlet 157 and into the annular space defined between the tube 176 and the sheath 190, and further directed through the outflow path further defined between the tube 176 and the transmission line 150. It is contemplated that the inflow path and the outflow path are provided in reverse in which the cooling liquid is directed through the fluid inlet 157 and into the annular space defined between the tube 176 and the sheath 190, and thereafter directed through the outflow path further defined between the tube 176 and the transmission line 150. It is further contemplated that certain features from the other implementations of the probe 34 may be provided, for example, the choke.

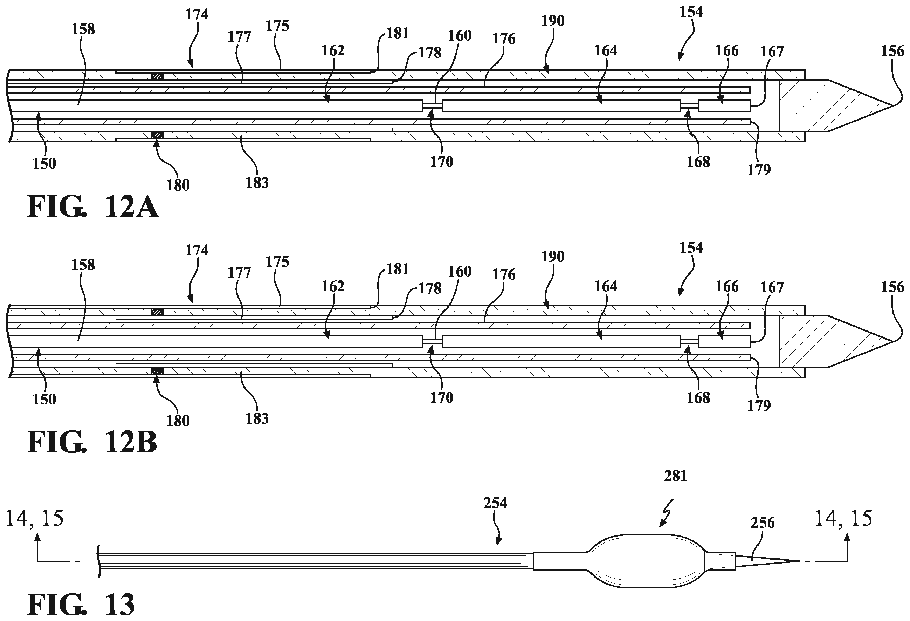

FIGS. 12A and 12B show alternative implementations of the probe 134 that include a choked, double-slot architecture with the choke 174 being secured to the sheath 190. The choke 174 may not be shorted to the outer conductor 158 of the transmission line 150. In other words, an inner choke layer 177 may be floating (*i.e.*, not grounded), and otherwise spaced apart from of the transmission line 150 to, optionally, provide for the closed-loop cooling system akin to that described with reference to FIG. 11. The transmission line 150 may include the proximal segment 162, the middle segment 164, and the distal segment 166. The inner conductor 160 may extend through the proximal segment 162 and the middle segment 164, and optionally the distal segment 166. The distal segment 166 and the middle segment 164 may define the first axial slot 168, and the middle segment 164 and the proximal segment 166 may define the second axial slot 170. Like numerals from FIG. 3 (plus one hundred) and from FIG. 11 identify like components, and corresponding disclosure is herein incorporated by reference.

The sheath 190 is a non-conductive tube coaxially disposed over a portion of the transmission line 150. In one example, the non-conductive tube is formed from fiberglass. The choke 174 includes the inner choke layer 177, an outer choke layer 175, and the electrical short 180. The inner choke layer 177 may be a first conductive tube coaxially disposed between the non-conductive tube and the outer conductor 158 of the transmission line 150. The first conductive tube may be a hypodermic tube of biocompatible metal. FIG. 12A represents a first variant in which the first conductive tube extends proximally from the active tip to the hub (not shown) to provide mechanical support and stiffness to the shaft 154 of the probe 134. The outer choke layer 175 may include a second conductive tube coaxially disposed over a portion the non-conductive tube such that a region 183 of the non-conductive tube forms a choke insulative layer. The electrical short 180 provides electrical communication between the inner choke layer 177 and the outer choke layer 175. FIG. 12B represents a second variant in which the second conductive tube forming the outer choke layer 175 extends proximally from the active tip to the hub to provide the mechanical support and stiffness to the shaft 154. In the second variant, the inner choke layer 177 may be one of a conductive tube extending proximally to the hub, a conductive tube segment as illustrated, or an electroplated layer to be described. In a third variant, each of the outer choke layer 175, the inner choke layer 177, and the electrical short 180 are formed from a continuous electroplated layer.

In certain implementations, the region 183 of the non-conductive tube is thinned to define a recess. The second conductive tube forming the outer choke layer 175 may be disposed within the recess. The arrangement provides for facilitating secure engagement of the second conductive tube and providing a smooth contour to the outer diameter of the shaft 154. A length of the region 183 is based on the relative permittivity of the material forming the sheath 190. In other words, the region 183 of the non-conductive tube may have a length approximately equal to one-quarter effective wavelength of a driving frequency in the material of the non-conductive tube. For example, the relative permittivity of the fiberglass may dictate the length of the choke 174, and in particular the region 183. The region 183 (*e.g.*, the thinned region) effectively becomes a waveguide with infinite impedance at a distal end of the choke 174 defined at a distal end 181 of the second conductive tube forming the outer choke layer 175.

In certain implementations, the electrical short 180 may be a conductive element that is compressed between the outer choke layer 175 and the inner choke layer 177. In one example, the conductive element is a wire or a ring. The conductive element may be formed from a suitably conductive material such as gold, or alternatively a solder may form the conductive element. Alternatively, the electrical short 180 may be formed through electroplating, as mentioned. The electrical short 180 may be optional, as it is contemplated that a choking effect may be provided between the inner and outer choke layers 175, 177 without a discrete component providing electrical communication therebetween. In variants in which the outer choke layer 175 is not electrically connected to the inner choke layer 175, a sleeve may be formed over the inner choke layer 177 with the sleeve having a length of a quarter of the wavelength of the driving frequency, or other integer multiples of this value.

With continued reference to FIGS. 12A and 12B, the distal end 178 of the inner choke layer 177 terminates at a position proximal to the second axial slot 170. Likewise, a distal end 181 of the outer choke layer 175 terminates at a position proximal to the second axial slot 170. The distance between the second axial slot 170 and one or both of the distal ends 178, 181 may be selectively tuned to impart the desired performance characteristics to the probe 134. In the illustrated implementations, the distal end 178 of the inner choke layer 177 is at a position distal to the distal end 181 of the outer choke layer 175. In another implementation, the distal ends 178, 181 of the inner and outer choke layers 175, 177 are coterminous. It should be appreciated that the first conductive tube forming the inner choke layer 177 may not be shorter than the second conductive tube forming the outer choke layer 175. In other words, the distal end 178 of the inner choke layer 177 may not be at a position proximal to the distal end 181 of the outer choke layer 175.

According to certain exemplary methods of assembly of the shaft 154 of the probe 34, the outer choke layer 175 is formed from the second conductive tube that extends and secured to the hub 152. The inner choke layer 177 is formed from an electroplated layer on the non-conductive tube or another dielectric member. The electroplated layer may be a segment or extend proximally to the hub 152. Alternatively, the outer choke layer 175, the inner choke layer 177, and the electrical short 180 are formed from a continuous electroplated layer on the non-conductive tube or another dielectric member. The electroplated tip assembly may be coupled to a hypodermic tube via a soldering process, and/or joined through adhesive or other suitable manufacturing technique. Such variants including electroplating may include the electroplated layer being at least two microns in thickness. Implementations including the electroplated layer may obviate the need for the recess of the non-conductive tube.

Another method of assembly includes plastically deforming of one or both of the first and second conductive tubes forming the inner choke layer 177 and the outer choke layer 175, respectively. For example, the plastic deformation may be facilitated by a swaging or crimping process in which the inner choke layer 177 and/or the outer choke layer 175 are plastically deformed into near contact or direct contact with one another, thereby forming the electrical short 180. Additional fastening means (e.g., solder, conductive adhesive, etc.) may be applied at the point of contact to improve conductivity of the electrical short 180.

The functional advantages of the distal end 181 of the choke 174 terminating at a position proximal to the second axial slot 170 are readily appreciated. As previously explained, the choke 174 is configured to prevent backward wave propagation along the shaft 154. The benefits of the choke 174, in and of itself, may not be fully realized across a range of relative permittivites of the tissue. For example, the effects of the choke 174 may be suboptimal in highly ablated tissue. The double-slot architecture, in combination with the choke 174, realizes pronounced synergy. The double-slot architecture focuses the energy at the appropriate location along the shaft 154 and accommodates highly ablated tissue. Taken together, the choke 174 is configured to prevent backwards current propagation initially in unablated (e.g., "raw" tissue), and the double-slot architecture is configured to constrain the field when the tissue is heavily ablated. The combination of the choke 174 and the double-slot architecture facilitates the near-spherical shape of the near-field region across an improved range of relative permittivites of the tissue as the tissue is being ablated.

In certain implementations, the probe 34, 134 may be configured to cool the transmission line 50, 150 with the cooling liquid, and discharge the infusion liquid into the tissue. In other words, the probe 34, 134 may be a hybrid of the above-described approaches in which at least a portion of the liquid is infused into the tissue, and the remainder is circulated to the pump 36 (or the liquid reservoir 38). A ratio of the liquid that is infused into the tissue to the liquid that is circulated may be fixed based on the characteristics of the probe 34, 134, for example, the size and/or number of the fluid ports 100, anticipated operating pressures, and filtering layers over the sheath 90, 190, and the like. Alternatively, the probe 34, 134 may include an infusion flow path that is fluidly separate from a cooling flow path. For example, the probe 34, 134 may include another hypodermic tube coaxially disposed within the tube 76, 176 and extending to near the tip 56, 156. The tube 76, 176 and the hypodermic tube define the inflow and outflow paths of the cooling flow path, in a manner similar to that for FIGS. 10 and 11, and an annular space between the tube 76, 176 and the sheath 90, 190 define the infusion flow path. The pump 36 may include valving, or valving may otherwise be provided, for the controller 44 to independently control the flow rate of the infusion liquid through the infusion flow path and the cooling liquid through the cooling flow path. The controller 44 may operate the system 30 in an infusion mode in which the infusion liquid is directed through the infusion flow path, but the cooling liquid being directed in the cooling flow path is suspended (e.g., the valving is selectively opened and closed). The controller 44 may operate the system 30 in a cooling mode in which the cooling liquid is directed through the cooling flow path, but the infusion liquid being directed the infusion flow path is suspended. Further, the controller 44 may operate the system 30 in a combination mode where the flow rates of the infusion liquid and the cooling liquid are independently controlled in a simultaneous manner. The selective operation of the system 30 in one of the infusion mode, the cooling mode, and the combination mode may be selected by the user on the display 104, or automatically initiated or terminated in real-time based on characteristics of the ablation procedure as determined in the manners previously described. Lastly, certain implementations of the probe 134 (e.g., FIGS. 10 and 11) may provide for an infusion-only mode in which separation of the inflow and outflow paths are not necessary, and therefore the tube 176 may be optional.

The probe 34, 134 and control aspects described herein may be used for tract ablation, that is, ablating during withdrawal of the probe 34, 134 from the tract through which the probe 34, 134 is directed. Tract ablation cauterizes the tissue and reduces the risk for tumour seeding. Yet the characteristics of the ablated tract are dependent on the technique of the surgeon. The system 30 of the present disclosure may provide for tract ablation in an automatic and consistent manner with no cognitive load on the surgeon. In certain implementations, the probe 34, 134 may include a temperature sensor along the shaft 54, 154 or another suitable location to sense the temperature of the tissue within the tract. The system 30 may be configured to ensure the temperature is sufficiently elevated to ensure cell death during the tract ablation. The pump 36 is operated to control the flow rate of the cooling fluid, as mentioned, and further may provide reversible flow. In such an arrangement, the cooling liquid may initially flow along the outflow path for portions of the ablation in which it is desired to cool the tissue, then reversed to permit the cooling liquid to be heated by the active antenna region of the shaft 54, 154. For example, an initial flow rate may be tuned so that the cooling fluid on the inflow path may heat the shaft 54, 154 rather than cool the shaft 54, 154. During the first part of the ablation, the cooling fluid is pumped by the pump 36 at a slow rate to keep the probe 34, 134 sufficiently cool to prevent damage, but otherwise hot relative to the tissue temperature as sensed by the temperature sensor. This allows heated cooling liquid to flow along the outflow path of the probe 34, 134. An outer wall of the shaft 54, 154 facilitates heat transfer, thereby providing a short period of time in which the heated cooling liquid transfers heat along the shaft 54, 154 to effectively perform automatic track ablation. Such an arrangement is particularly well suited for the double slot architectures of the probe 34, 134 described herein in which the tissue more rapidly reaches the ablated stage an in which the probe 34, 134 becomes self-choked. When the probe 34, 134 reaches a self-choked condition, the controller 44 may operate the pump 36 to ramp up the flow rate. The increase in the flow rate of the heated cooling liquid facilitates maintaining the self-choked condition and terminates the automatic tract ablation to prevent excessive damage to the tissue surrounding the probe 34, 134.

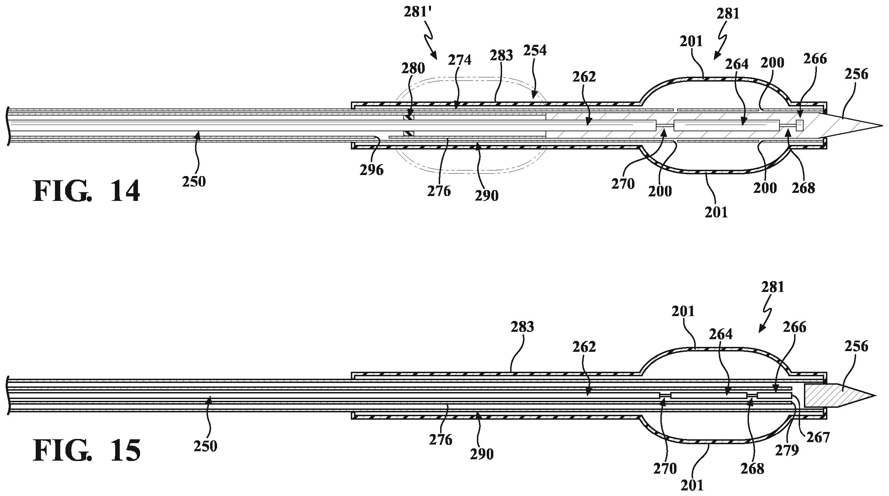

Referring now to FIGS. 13-15, variants of the shaft 254 of the double-slot architecture are shown in which the probe includes an inflatable member 281. The inflatable member 281 may be coupled to the dielectric housing 284 and in fluid communication with at least the inflow path. For example, FIG. 14 shows the inflatable member 281 in fluid communication with the distal infusion path through the fluid ports 200 defined in the sheath 190. Therefore, the inflatable member 281 is in fluid communication with the proximal infusion path through aperture 196 defined by the tube 176 that provides fluid communication about the choke 174 as previously described. A sleeve 283 may be coaxially disposed over the sheath 190, and the inflatable member 281 may be coupled to the sleeve 283 at any suitable axial position so as to achieve the desired ablation zone. In the illustrated example, the inflatable member 281 is axially positioned near the first and second slots 168, 170, which may be particularly advantageous to localize a volume of liquid near the location of maximum microwave energy transfer.

The inflatable member 281 may be formed from non-compliant material. Doing so prevents undue expansion during inflation, from which the volume of the liquid within the inflatable member 281 can be reliably known. The inflation of the inflatable member 281 advantageously engages the tissue to limit or eliminate "pull out" of the probe from the tissue, for example, with excessive jostling. Further, as mentioned, localizing the volume of liquid within the ablation zone may provide for improved control over the shape of the ablation. The inflatable member 281 may be spherical or oblong, as generally shown in FIGS. 13-15, or eccentric shapes may be utilized to facilitate a desired heating pattern adjacent to the active tip. It is further contemplated that multiple inflatable members may be disposed along the shaft 254. For example, FIG. 14 shows a second inflatable member 281' (in phantom) axially positioned about the choke 274. In one variant the second inflatable member 281' is included, and the inflatable member 281 is optional.

The inflatable member 281 may include fenestrations, perforations, or other suitably small orifices configured to facilitate weeping of the infusion liquid into the tissue. Owing to the quantity and/or size of the fenestrations (*e.g.*, porosity), the infusion liquid maintains the inflatable member 281 in an inflated state. The size and/or quantity of the fenestrations may be specifically designed to permit infusion of the infusion liquid at a desired flow rate while also maintaining the inflatable member 281 at a minimum or predetermined pressure. Additionally or alternatively, microporosity of the inflatable member 281 may be provided by the inflatable member 281 becoming fully inflated or stretching slightly. The infusion liquid weeping from the inflatable member 281 and into the adjacent tissue may be heated by the microwaves and generate steam. As mentioned, the steam may advantageously provide for an additional heat transfer mechanism. The generation of steam may be further facilitated by the preheating of the infusion liquid, which may be realized in the proximal and distal infusion paths in the arrangement of FIG. 14. Should any of the infusion liquid vaporize within the inflatable member 281, the vapor may merely expel through the fenestrations and/or microporosity without issue.

FIG. 15 shows another variant of the shaft 254 of the probe in which the inflow and outflow paths for cooling are provided. The tube 276 is coaxially positioned within the sheath 290 and has its distal end 279 axially positioned coterminous (or proximal or distal to) with the distal end 267 of the transmission line 250. The cooling liquid is directed through the annular space defined between the tube 276 and the sheath 290, and further directed through the outflow path further defined between the tube 276 and the transmission line 250 (or the reverse). The inflatable member 281 is coupled to the sleeve 283 which defines with the sheath 290 an inflation flow path therebetween. The inflation flow path may be arranged in fluid communication with the inflow path from which it receives a portion of its fluid, or the inflation flow path may be fluidly separate from the inflow path. The controller 44 may operate the pump 36 (and/or a second pump) to independently control the flow rate of the inflation liquid and the cooling liquid in a simultaneous manner. In another implementation, the inflatable member 281 includes the fenestrations, perforations, microporosity, or the like, in which the inflation flow path may be considered the infusion flow path. The controller 44 may operate the system 30 in the cooling mode, the infusion mode, the combination mode, or an inflation mode in which the inflatable member 281 is inflated and other fluid transport is suspended.

Certain inventive aspects of the present disclosure are appreciated with reference to the following exemplary clauses.

Clause 1 - A probe for ablating tissue with microwave energy, the probe comprising: a transmission line comprising an outer conductor, an inner conductor coaxially disposed within the outer conductor, and an insulative layer coaxially disposed between the outer conductor and the inner conductor; a dielectric housing coupled to the transmission line and defining a distal tip of the probe; a sheath coaxially disposed over the outer conductor and at least a portion of the dielectric housing, the sheath defining an infusion path configured to receive infusion liquid; and an inflatable member coupled to the dielectric housing and in fluid communication with the infusion path and defines micropores or fenestrations, wherein the inflatable member is configured to be inflated with the infusion liquid for the infusion liquid to weep into the tissue through the micropores or fenestrations.

Clause 2 - The probe of clause 1, further comprising any embodiment of the choke and described and disclosed herein.

Clause 3 - The probe of clause 1, further comprising any embodiment of the choke and described and disclosed herein.

Clause 4 - A method of performing an ablation procedure in which a probe is coupled a generator to ablate tissue and to an analyzer, the method comprising: delivering microwave energy to the probe to ablate the tissue; determining a measured S11 parameter based on reflected power sensed by the generator at a driving frequency; performing a frequency sweep in which a sensed S11 parameter is determined for at least two frequencies; determining a characteristic of the ablation procedure based on the measured S11 parameter and the frequency sweep; and at least one of (i) controlling delivery of an infusion liquid, (ii) controlling flow rate of a cooling liquid, and (iii) controlling delivery of the microwave energy based on the determined characteristic.

Clause 5 - The method of clause 4, further comprising evaluating the sensed S11 parameters against calibration data for the probe across a range of frequencies at different known values of average relative permittivity.

Clause 6 - The method of clause 5, wherein the calibration data further includes a plurality of power curves, each of the power curves being associated with one of the different known values of average relative permittivity, the method further comprising selecting one of power curves based on the sensed S11 parameters.

Clause 7 - The method of clause 6, wherein each of the power curves includes a resonant frequency, the method further comprising: determining a slope of datapoints associated with the sensed S11 parameters, wherein the slope is either positive or negative; and determining whether the resonant frequency is greater or less than the driving frequency based on the slope.

Clause 8 - The method of clause 7, further comprising initiating, increasing or decreasing the infusion liquid provided to the tissue if the measured resonant frequency is less than the driving frequency and if the determined slope is positive.

Clause 9 - The method of clause 8, further comprising initiating or increasing the infusion liquid to the tissue by an amount proportional to a difference between the resonant frequency and the driving frequency.

Clause 10 - The method of clause 7, further comprising maintaining, decreasing, or terminating the infusion liquid being delivered to the tissue if the measured S11 parameter is equal to a designed resonant frequency of the probe.

Clause 11 - The method of any one of clauses 4-10, wherein the determined characteristic is weighted average relative permittivity.

Clause 12 - The method of any one of clauses 4-11, further comprising: transforming the frequency sweep from a frequency domain spectrum to a time domain spectrum; determining average relative permittivity of the tissue; determining a change in speed of the microwaves with the changes being indicative of changes in electrical properties of the tissue; and providing on a display an indication as to a characteristic or status of the ablation procedure.

Clause 13 - A method of determining a characteristic of an ablation procedure in which a probe is coupled a generator to ablate tissue, the method comprising: delivering microwave energy to the probe to ablate the tissue; performing a frequency sweep; transforming the frequency sweep from a frequency domain spectrum to a time domain spectrum; determining speed of the microwaves based on sensed times by which microwaves are reflected the tissue; determining a change in speed of the microwaves with the changes being indicative of changes in electrical properties of the tissue; and providing on a display an indication as to a characteristic or status of the ablation procedure.

Clause 14 - The method of clause 13, further comprising providing on the display an alert that the probe has passed through an interface between a lesion and adjacent tissue.

Clause 15 - The method of clause 14, further comprising: determining a change in average relative permittivity based on the frequency sweep, wherein the speed of the microwaves is based on the average relative permittivity; and compensating for the change in the average relative permittivity in determining the change in speed of the microwaves.

Clause 16 - The method of any one of clauses 13-15, further comprising: determining distances to reflection points associated with the interface based on the speed of the microwaves; estimating lesion size based on the determined distances; and providing on the display a virtual representation of the lesion size.

Clause 17 - A method of determining a characteristic of an ablation procedure in which a probe is coupled a generator to ablate tissue, the method comprising: delivering microwave energy to the probe to ablate the tissue; performing a frequency sweep in a frequency domain spectrum; determining an average relative permittivity based on the frequency sweep; and transforming the frequency sweep from the frequency domain spectrum to a time domain spectrum; determining an absence or presence of reflection points in the time domain spectrum; determining distances to the reflection points based on speed of the microwaves; comparing the distance against a previously determined distance; and providing on a display an indication of a boundary between ablated and non-ablated tissue if the distances are indicative that the reflection points are new reflection points.

Clause 18 - The method of any one of clauses 4-17, wherein the step of delivering the microwave energy to ablate the tissue is performed in a high-power mode of a first wattage, and the step of performing the frequency sweep is performed at a low-power mode of a second wattage that is less than the first wattage.

Clause 19 - The method of any one of clauses 4-18, wherein the generator includes a controller, and a pump in communication with the controller, the method further comprising operating the pump to control the delivery of the infusion liquid.

Clause 20 - An instrument console configured to be removably coupled with a probe to perform any one of the methods of clauses 4-19, the instrument console comprising: a microwave generator configured to generate microwave energy at a first wattage; a vector network analyzer configured to generate microwave energy at a second wattage less than the first wattage and to perform analysis in a frequency domain spectrum and a time domain spectrum; a switch configured to selectively establish communication between the microwave generator and one of the probe and the vector network analyzer; and a controller in communication with the microwave generator and the switch, wherein the controller is configured to control the switch to establish communication between the microwave generator and the probe to ablate tissue in a high-power mode at the first wattage, or establish communication between the probe and the vector network analyzer to perform a frequency sweep in a low-power mode at the second wattage.

Clause 21 - The instrument console of clause 20, further comprising a pump in communication with the controller, wherein the controller is configured to operate the pump based on data received from the vector network analyzer.

Clause 22 - The instrument console of clause 20 or 21, further comprising the probe of any one of clauses 1-3, and/or any of the embodiments of the probe described and disclosed herein.

The foregoing disclosure is not intended to be exhaustive or limit the invention to any particular form. The terminology which has been used is intended to be in the nature of words of description rather than of limitation. Many modifications and variations are possible in light of the above teachings and the invention may be practiced otherwise than as specifically described.
The present disclosure also provides for the following examples:
1. A probe for ablating tissue with microwave energy, the probe comprising:
   a transmission line comprising an outer conductor, an inner conductor coaxially disposed within the outer conductor, an insulative layer coaxially disposed between the outer conductor and the inner conductor, wherein the transmission line defines a first axial slot and a second axial slot located proximal to the first axial slot;
   a non-conductive tube coaxially disposed over a portion of the transmission line;
   an electrical choke coupled to the non-conductive tube and comprising:
      an inner choke layer comprising a first conductive tube coaxially disposed between the non-conductive tube and the outer conductor of the transmission line, wherein the inner choke layer has a distal end that terminates at a position proximal to the second axial slot;
      an outer choke layer comprising a second conductive tube coaxially disposed over the non-conductive tube such that a region of the non-conductive tube forms a choke insulative layer, wherein the outer choke layer has a distal end that terminates at a position proximal to the second axial slot; and
      an electrical short providing electrical communication between the inner choke layer and the outer choke layer.
2. The probe of example 1, wherein the non-conductive tube defines a recess, wherein the second conductive tube coaxially disposed within the recess.
3. The probe of example 1 or 2, wherein the distal end of the inner choke layer is distal to the distal end of the outer choke layer.
4. The probe of example 1 or 2, wherein the distal end of the inner choke layer and the distal end of the outer choke layer are coterminous.
5. A probe for ablating tissue with microwave energy, the probe comprising:
   a transmission line comprising an outer conductor, an inner conductor coaxially disposed within the outer conductor, an insulative layer coaxially disposed between the outer conductor and the inner conductor;
   a non-conductive tube coaxially disposed over a portion of the transmission line, wherein a region of an outer surface the non-conductive tube defines a recess;
   an electrical choke coupled to the non-conductive tube and comprising:
      an inner choke layer comprising a first conductive tube coaxially disposed between the non-conductive tube and the outer conductor of the transmission line;
      an outer choke layer comprising a second conductive tube disposed within the recess such that a portion of the non-conductive tube forms a choke insulative layer; and
      an electrical short providing electrical communication between the inner choke layer and the outer choke layer.
6. The probe of any one of examples 1-5, wherein the electrical short is formed through plastic deformation of and direct contact between the first conductive tube and the second conductive tube; and, optionally, wherein the plastic deformation is through a swaging process.
7. The probe of example 6, further comprising a solder or conductive adhesive at a point of the direct contact between the first conductive tube and the second conductive tube.
8. The probe of any of examples 1-7, wherein the electrical short is a conductive element that is compressed between the inner choke layer and the outer choke layer.
9. The probe of example 8, wherein the conductive element is a wire or a ring.
10. The probe of example 9, wherein the conductive element is formed of gold or solder.
11. A probe for ablating tissue with microwave energy, the probe comprising:
   a transmission line comprising an outer conductor, an inner conductor coaxially disposed within the outer conductor, an insulative layer coaxially disposed between the outer conductor and the inner conductor, wherein the transmission line defines a first axial slot and a second axial slot located proximal to the first axial slot;
   a non-conductive tube coaxially disposed over a portion of the transmission line;
   an electrical choke coupled to the non-conductive tube and comprising:
      an inner choke layer coaxially disposed between the non-conductive tube and the outer conductor of the transmission line;
      an outer choke layer coaxially coupled to an outer surface of the non-conductive tube such that a region of the non-conductive tube forms a choke insulative layer, wherein the outer choke layer has a distal end that terminates at a position proximal to the second axial slot; and
      an electrical short providing electrical communication between the inner choke layer and the outer choke layer,
   wherein at least one of the inner choke layer, the outer choke layer, and the electrical short are formed through conductive foil and/or electroplating.
12. The probe of example 11, wherein the inner choke layer, the outer choke layer, and the electrical short are formed through a continuous electroplated layer to form an electroplated tip assembly with the non-conductive tube; and, optionally, wherein the electroplated tip assembly is secured to a hypodermic tube.
13. The probe of any of examples 1-12, wherein the region of the non-conductive tube forming the choke insulative layer has a length approximately equal to one-quarter effective wavelength of a driving frequency in a material forming the non-conductive tube.
14. The probe of any one of examples 1-13, further comprising an additional non-conductive tube coaxially disposed between the transmission line and the inner choke layer, wherein the additional non-conductive tube defines an inflow fluid path and an outflow fluid path.
15. The probe of example 14, wherein a distal end of the additional non-conductive tube is axially positioned proximal to a distal end of the transmission line.
16. The probe of example 14, wherein a distal end of the additional non-conductive tube and a distal end of the transmission line are coterminous.
17. A probe for ablating tissue with microwave energy, the probe comprising:
   a transmission line comprising an outer conductor, an inner conductor coaxially disposed within the outer conductor, an insulative layer coaxially disposed between the outer conductor and the inner conductor;
   a non-conductive tube coaxially disposed over a portion of the transmission line;
   an electrical choke coupled to the non-conductive tube and comprising:
      an inner choke layer comprising a first conductive tube coaxially disposed between the non-conductive tube and the outer conductor of the transmission line, wherein the inner choke layer is spaced apart from the outer conductor of the transmission line; and
      an outer choke layer comprising a second conductive tube such that a region of the non-conductive tube forms a choke insulative layer.
18. The probe of example 17, further comprising an electrical short providing electrical communication between the inner choke layer and the outer choke layer.
19. A probe for ablating tissue with microwave energy, the probe comprising:
   a transmission line comprising an outer conductor, an inner conductor coaxially disposed within the outer conductor, and an insulative layer coaxially disposed between the outer conductor and the inner conductor;
   an electrically conductive tube coaxially disposed over a portion of the transmission line, wherein an electrical short provides electrical communication between the tube and the outer conductor of the transmission line to define a choke in which a choke insulative layer is disposed between the outer conductor and the tube, wherein the tube defines a proximal infusion path configured to be arranged in fluid communication with a source of infusion liquid, and an aperture; and
   a sheath coaxially disposed over the tube, wherein the sheath defines a distal infusion path in fluid communication path with the proximal infusion path through the aperture, and fluid ports in fluid communication with the distal infusion path.
20. The probe of example 19, wherein the aperture is located proximal to the electrical short such that at least a portion of the transmission line is cooled with the infusion liquid prior to passing through the aperture.
21. The probe of example 19 or 20, wherein the proximal infusion path is further defined between the tube and the outer conductor.
22. The probe of any one of examples 19-21, wherein the fluid ports are localized to an active tip of the probe axially defined between the choke and the distal end of the tip of the probe.
23. The probe of example 22, wherein the fluid ports are uniformly spaced along a length of the active tip.
24. The probe of any one of examples 19-23, wherein the fluid ports are uniformly distributed radially about the sheath.
25. The probe of any one of example 19-24, wherein a distal fluid port is defined at a distal end of the sheath.
26. The probe of any one of examples 19-25, wherein the sheath is polymeric.
27. The probe of example 26, wherein the fluid ports are micropores within the polymeric sheath.
28. The probe of any one of examples 19-27, further comprising an end cap coupled to the inner conductor and defining a first axial slot the outer conductor of the transmission line, wherein the fluid ports are positioned proximal to the first axial slot.
29. The probe of example 28, wherein the outer conductor of the transmission line further defines a second axial slot positioned proximal to the first axial slot, wherein the first axial slot and the second axial slot are spaced apart by a middle segment of the outer conductor that is one-quarter effective wavelength of the tissue.
30. A probe for ablating tissue with microwave energy, the probe comprising:
   a transmission line comprising an outer conductor, an inner conductor coaxially disposed within the outer conductor, an insulative layer coaxially disposed between the outer conductor and the inner conductor, wherein the transmission line defines a first axial slot and a second axial slot located proximal to the first axial slot;
   a tube coaxially disposed over a portion of the transmission line, wherein an electrical short provides electrical communication between the tube and the outer conductor of the transmission line to define a choke in which a choke insulative layer is disposed between the outer conductor and the tube; and
   a sheath coaxially disposed over the tube and defining an infusion path configured to be arranged in fluid communication with a source of infusion liquid, and further defining fluid ports positioned between the first axial slot and the second axial slot.
31. The probe of example 30, further comprising an end cap coupled to the inner conductor, wherein the first axial slot is defined between the end cap and the outer conductor.
32. The probe of example 31, wherein the second axial slot is positioned proximal to the fluid ports.
33. The probe of any one of examples 30-32, wherein there is a higher concentration of the fluid ports positioned between the first axial slot and the second axial slot than either distal to the first axial slot or proximal to the second axial slot.
34. The probe of any one of examples 30-33, wherein there is a higher concentration of the fluid ports positioned adjacent to the choke than distal to the choke.
35. The probe of any one of examples 30-34, wherein the first axial slot and the second axial slot are each approximately one millimeter in length.
36. The probe of any one of examples 30-35, wherein a transmission line further comprises a proximal segment of the outer conductor that is positioned distal to the choke, wherein a length of the proximal segment is one-quarter effective wavelength of the tissue.
37. The probe of any one of examples 30-36, further comprising a dielectric housing at least partially disposed within the sheath, wherein a choke length of the choke defined between the electrical short and an interface between the outer conductor and the dielectric housing is configured to be one-quarter effective wavelength of a microwave field in the choke insulative layer.
38. The probe of any one of examples 30-37, further comprising a dielectric housing at least partially disposed within the sheath, and an inflatable member coupled to the dielectric housing and in fluid communication with the infusion path, wherein the inflatable member is configured to be inflated with the infusion liquid to prevent movement of the probe within the tissue.
39. The probe of any one of examples 30-38, further an inflatable member coupled to the sheath to define an inflation flow path, wherein the probe defines an inflow path and an outflow path that are fluidly separate from the inflation flow path configured to circulate cooling fluid.

## Claims

1. A probe (34) for ablating tissue with microwave energy, the probe (34) comprising:
a transmission line (50) comprising an outer conductor (58), an inner conductor (60) coaxially disposed within the outer conductor (58), and an insulative layer coaxially disposed between the outer conductor (58) and the inner conductor (60);
an electrically conductive tube (76) coaxially disposed over a portion of the transmission line (50), wherein an electrical short (80) provides electrical communication between the tube (76) and the outer conductor (58) of the transmission line (50) to define a choke (74) in which a choke insulative layer (82) is disposed between the outer conductor (58) and the tube (76), wherein the tube (76) defines a proximal infusion path (94) configured to be arranged in fluid communication with a source of infusion liquid (38), and an aperture (96); and
a sheath (90) coaxially disposed over the tube (76), wherein the sheath (90) defines a distal infusion path (92) in fluid communication path with the proximal infusion path (94) through the aperture (96), and fluid ports (100) in fluid communication with the distal infusion path (92).

2. The probe (34) of claim 1, wherein the aperture (96) is located proximal to the electrical short (80) such that at least a portion of the transmission line (50) is cooled with the infusion liquid prior to passing through the aperture (96).

3. The probe (34) of claim 1 or 2, wherein the proximal infusion path (94) is further defined between the tube (76) and the outer conductor (58).

4. The probe (34) of any one of claims 1-3, wherein the fluid ports (100) are localized to an active tip of the probe (34) axially defined between the choke (74) and a distal end of a tip (56) of the probe (34).

5. The probe (34) of claim 4, wherein the fluid ports (100) are uniformly spaced along a length of the active tip.

6. The probe (34) of any one of claims 1-5, wherein the fluid ports (100) are uniformly distributed radially about the sheath (90).

7. The probe (34) of any one of claim 1-6, wherein a distal fluid port (102) is defined at a distal end of the sheath (90).

8. The probe (34) of any one of claims 1-7, wherein the sheath (90) is polymeric.

9. The probe (34) of claim 8, wherein the fluid ports (100) are micropores within the polymeric sheath (90).

10. The probe of any one of claims 1-9, further comprising an end cap (66) coupled to the inner conductor (60) and defining a first axial slot (68) of the outer conductor (58) of the transmission line (50), wherein the fluid ports (100) are positioned proximal to the first axial slot (68).

11. The probe (34) of claim 10, wherein the outer conductor (58) of the transmission line (50) further defines a second axial slot (70) positioned proximal to the first axial slot (68), wherein the first axial slot (68) and the second axial slot (70) are spaced apart by a middle segment (64) of the outer conductor (58) that is one-quarter effective wavelength of the tissue.

12. The probe (34) of claim 11, wherein the fluid ports (100) are positioned between the first axial slot (68) and the second axial slot (70).

13. The probe (34) of claim 12, wherein there is a higher concentration of the fluid ports (100) positioned between the first axial slot (68) and the second axial slot (70) than either distal to the first axial slot (68) or proximal to the second axial slot (70).

14. The probe (34) of any one of claims 1-13, wherein there is a higher concentration of the fluid ports positioned adjacent to the choke (74) than distal to the choke (74).

15. The probe (34) of any one of claims 1-14, wherein the transmission line (50) further comprises a proximal segment (62) of the outer conductor (58) that is positioned distal to the choke (74), wherein a length of the proximal segment (62) is one-quarter effective wavelength of the tissue.
